# EUROPEAN PATENT APPLICATION

(11) **EP 4 779 750 A1**
(43) Date of publication of application: **22.07.2026**
(21) Application number: 25864508.4
(22) Date of filing: 30.05.2025
(51) Int. Cl.: H01M 10/42, G01N 33/00

(54) **APPARATUS FOR INSPECTING BATTERY CELL**

(30) Priority: 19.11.2024 KR 20240165117
(71) Applicant: LG ENERGY SOLUTION, LTD., Seoul 07335 (KR)
(72) Inventor: PARK, Yang Kyu, Daejeon, 34122 (KR); CHO, Young Jun, Daejeon, 34122 (KR); JI,Yu Jeong, Daejeon, 34122 (KR); CHO, Hyun Jun, Daejeon, 34122 (KR)
(74) Representative: Goddar, Heinz J.
(86) International application number: PCT/KR2025/007459
(87) International publication number: WO 2026/111065

(57) **Abstract**

The present disclosure relates to an apparatus for inspecting a battery cell including a battery cell tray in which a plurality of battery cells are seated; and a sensing unit that simultaneously senses leak gas leaking from the plurality of battery cells individually for each battery cell, thereby obtaining the advantageous effect of simply and accurately testing whether a battery cell leaks.

## Description

### TECHNICAL FIELD

### CROSS-REFERENCE TO RELATED APPLICATIONS

The present application is based on and claims priority from Korean Patent Application No. 10-2024-0165117, filed on November 19, 2024, with the Korean Intellectual Property Office, the disclosure of which is incorporated herein in its entirety by reference.

### TECHNICAL FIELD

The present disclosure relates to an apparatus for inspecting a battery cell, and more specifically, to an apparatus for inspecting a battery cell capable of simply and accurately testing whether a battery cell leaks.

### BACKGROUND ART

With the development of technology in electric vehicles, energy storage systems (ESS), and portable electronic devices, the demand for a lithium secondary battery as an energy source is rapidly increasing.

A lithium secondary battery may be classified into a pouch type and a can type depending on the material of a case (exterior material) that accommodates an electrode assembly, and the electrode assembly may be classified into a wound type (jelly roll type), a stacked type (stack type), a stack and lamination type, or a stack and folding type, depending on the manufacturing method and shape.

Among these, the jelly roll type electrode assembly has a structure in which a positive electrode and a negative electrode formed by applying an electrode active material slurry to a positive electrode current collector and a negative electrode current collector are wound in a roll shape together with a separator, and is widely used due to its advantages of being easy to manufacture and having high energy density per unit weight.

Meanwhile, since electrolyte leakage from a battery cell not only reduces the performance and life of the battery cell but also increases the risk of explosion, it is necessary to conduct a preliminary test on the sealing performance (electrolyte leakage) of the battery cell.

For example, in the case of a cylindrical can-type battery cell, if a welding defect (e.g., over-welding or crack) occurs in the cap covering the opening of the case that accommodates the electrode assembly, the electrolyte may leak through the welding area of the cap.

To this end, various studies have been conducted recently to test the sealing performance of battery cells, but no fundamental measures have yet been established.

In particular, there has been no measures established to select defective products (defective battery cells that leak electrolyte) among the plurality of battery cells while simultaneously performing sealing tests on the plurality of battery cells, and development of these measures is urgently required.

### SUMMARY

### TECHNICAL PROBLEM

The embodiment of the present disclosure is directed to providing an apparatus for inspecting a battery cell capable of simply and accurately testing whether a battery cell leaks.

In particular, the embodiment of the present disclosure is directed to selecting defective products among the plurality of battery cells while simultaneously performing sealing tests on the plurality of battery cells using a non-destructive inspection method.

Additionally, the embodiment of the present disclosure is directed to simplifying the process of inspecting the sealing performance of a battery cell and improving the accuracy and reliability of the inspection.

Additionally, the embodiment of the present disclosure is directed to enabling an operator to quickly and accurately inspect whether a battery cell leaks on the site without using expensive equipment or relying heavily on the experience and capability of the operator.

Additionally, the embodiment of the present disclosure is directed to shortening the time required to test the sealing performance of a battery cell and reducing the cost.

The problem to be solved in the embodiment is not limited thereto, and it can be said that the purpose or effect that may be identified from the method of solution or embodiment of the problem described below is also included.

### TECHNICAL SOLUTION

According to a preferred embodiment of the present disclosure for achieving the objects of the present disclosure described above, an apparatus for inspecting a battery cell includes a battery cell tray in which a plurality of battery cells are seated; and a sensing unit that simultaneously senses leak gas leaking from the plurality of battery cells individually for each battery cell.

This is to simply and accurately test the sealing performance of the battery cell.

That is, since electrolyte leakage from the battery cell not only reduces the performance and life of the battery cell but also increases the risk of explosion, it is necessary to conduct a preliminary test on the sealing performance (electrolyte leakage) of the battery cell. However, in the past, in order to determine whether the battery cell leaks, it was necessary to test the sealing performance of the battery cell within a separate vacuum chamber supplied with helium gas, which resulted in problems of significant time and cost expenditures in the process of inspecting whether the battery cell leaked.

Furthermore, in the past, it was necessary to prepare separate equipment for suppling helium gas inside the vacuum chamber, which caused a problem of increasing manufacturing cost and made it difficult to select defective products among a plurality of battery cells.

However, the embodiment of the present disclosure may obtain the advantageous effect of simplifying the sealing performance inspection process of the battery cell and improving inspection accuracy and reliability by simultaneously sensing leak gas leaking from the plurality of battery cells individually for each battery cell via a sensing unit.

Above all, the embodiment of the present disclosure may quickly and accurately determine whether a plurality of battery cells leak by selecting defective products among the plurality of battery cells while simultaneously performing sealing tests on the plurality of battery cells, and may obtain the advantageous effect of simplifying the structure of the inspection apparatus and the inspection process.

The battery cell tray may be provided in various structures in which a plurality of battery cells may be seated.

According to a preferred embodiment of the present disclosure, the battery cell tray may include a tray body; and a partition portion provided in the tray body and dividing an internal space of the tray body into a plurality of seating spaces where the plurality of battery cells are individually seated.

The partition portion may be provided in various structures capable of dividing the internal space of the tray body into a plurality of seating spaces.

According to a preferred embodiment of the present disclosure, the partition portion may include a first partition member dividing the internal space along a first direction of the tray body; and a second partition member dividing the internal space along a second direction intersecting the first direction and defining the seating space in cooperation with the first partition member.

According to a preferred embodiment of the present disclosure, the apparatus for inspecting a battery cell may include a support rib provided on an inner wall surface of the seating space and supporting the battery cell with respect to the tray body.

According to a preferred embodiment of the present disclosure, the apparatus for inspecting a battery cell may include a slope guide portion provided at an upper end of the support rib and guiding a lower end of the battery cell to the seating space.

In this way, according to the embodiment of the present disclosure, it is possible for the battery cell to more smoothly enter the interior of the seating space by providing a slope guide portion at the upper end of the support rib. Additionally, according to the embodiment of the present disclosure, the lower end of the battery cell is guided into the guide space along the slope guide portion, so that even if the center of the battery cell and the center of the buffer space are misaligned, the center of the battery cell may be coaxially guided (aligned) to the center of the buffer space, thereby minimizing misinsertion and misalignment of the battery cell and obtaining the advantageous effect of improving inspection efficiency.

According to a preferred embodiment of the present disclosure, the sensing unit is configured to sense the leak gas in an atmospheric pressure environment.

In this way, according to the embodiment of the present disclosure, the sensing unit is configured to sense the leak gas in an atmospheric pressure environment, so that it is not necessary to additionally provide a separate vacuum chamber and equipment for forming a vacuum environment, thereby obtaining the advantageous effect of simplifying the structure and inspection process and reducing the cost.

The sensing unit may be provided in various structures capable of individually and simultaneously sensing the leak gas leaking from each battery cell.

According to a preferred embodiment of the present disclosure, the sensing unit may include a sensor board; a sensing element provided on the sensor board and sensing the leak gas; and a leak gas guide provided between the sensor board and the battery cell tray and individually guiding the leak gas leaking from the battery cell to the sensing element.

The leak gas guide may be provided in various structures capable of individually guiding the leak gas leaking from each battery cell to the sensing element (sensing element group).

According to a preferred embodiment of the present disclosure, the leak gas guide may include a guide plate provided between the sensing element and the battery cell tray; and a guide hole formed through the guide plate and individually guiding the leak gas to the sensing element for each battery cell.

According to a preferred embodiment of the present disclosure, the apparatus for inspecting a battery cell may include a guide rib provided on a bottom surface of the guide plate facing the battery cell tray and defining a receiving space in which an upper end of the battery cell is individually accommodated.

In this way, according to the embodiment of the present disclosure, a guide rib is provided on the bottom surface of the guide plate, and the upper end of the battery cell is accommodated in the receiving space of the guide rib, so that the leak gas leaking from the battery cell may be concentrated into the guide hole without being scattered to the surroundings, thereby obtaining the advantageous effect of further improving the sensing performance and accuracy of the leak gas.

According to a preferred embodiment of the present disclosure, the apparatus for inspecting a battery cell may include a stopper portion provided on the guide plate and supporting the battery cell to be spaced apart from the sensing element.

This is because, when the electrolyte leaking from the battery cell is in direct contact with the sensing element, the sensing element becomes contaminated by the electrolyte, making it difficult for the sensing element to perform the inspection process normally.

However, according to the embodiment of the present disclosure, a stopper portion is provided on the guide plate, and the battery cell is supported to be spaced apart from the sensing element via the stopper portion, so that even if the electrolyte leaks from the battery cell, the electrolyte may be prevented from directly contacting the sensing element, thereby obtaining the advantageous effect of minimizing the deterioration of inspection accuracy and reliability due to contamination of the sensing element.

The stopper portion may be provided in various structures capable of supporting the battery cell to be spaced apart from the sensing element.

According to a preferred embodiment of the present disclosure, the stopper portion may be configured to support the edge end of the battery cell along the circumferential direction of the battery cell.

According to a preferred embodiment of the present disclosure, the apparatus for inspecting a battery cell may include a gasket member provided between the sensor board and the leak gas guide and independently sealing the sensing element corresponding to the battery cell.

The gasket member may be provided in various structures capable of independently sealing the sensing element (or sensing element group) between the sensor board and the leak gas guide.

According to a preferred embodiment of the present disclosure, the gasket member may include a gasket sheet interposed between the sensor board and the guide plate; and a through hole formed through the gasket sheet to communicate with the guide hole.

In this way, according to the embodiment of the present disclosure, each sensing element (or sensing element group) may be independently sealed via the gasket member, thereby obtaining the advantageous effect of minimizing malfunction (false sensing) of the sensing element due to leak gas introduced from other guide holes in the vicinity and improving stability and reliability.

According to a preferred embodiment of the present disclosure, the apparatus for inspecting a battery cell may include a sensor plate supporting the sensor board, wherein the sensor plate may be seated on the guide plate.

In this way, according to the embodiment of the present disclosure, the sensor board may be supported by the sensor plate, thereby obtaining the advantageous effect of more stably supporting the arrangement state of the sensor board with respect to the guide plate while ensuring the structural rigidity of the sensor board.

According to a preferred embodiment of the present disclosure, the apparatus for inspecting a battery cell may include a support member provided above the sensing unit and selectively supporting the sensing unit to allow it to move up and down.

According to a preferred embodiment of the present disclosure, the apparatus for inspecting a battery cell may include a control board provided on the support member and outputting sensing values sensed by the sensing unit for each battery cell to the outside.

According to a preferred embodiment of the present disclosure, the apparatus for inspecting a battery cell may include a sealing member provided between the guide rib and the partition portion and sealing the receiving space and the seating space for each battery cell.

In this way, according to the embodiment of the present disclosure, the gap between the guide rib and the partition portion may be sealed via the sealing member, so that it is possible to block the leakage of leak gas leaking from the battery cell into the gap between the receiving space and the seating space (the gap between the guide rib and the partition portion) or the flow of contaminated air or gas from the outside into the gap between the receiving space and the seating space, thereby obtaining the advantageous effect of minimizing the deterioration of the inspection accuracy and reliability of the sensing element due to the detection amount of leak gas falling below a reference value or the inflow of contaminated air.

### ADVANTAGEOUS EFFECTS

As described above, according to the embodiment of the present disclosure, it is possible to obtain the advantageous effect of simply and accurately testing whether a battery cell leaks.

In particular, according to the embodiment of the present disclosure, it is possible to obtain the advantageous effect of selecting defective products among the plurality of battery cells while simultaneously performing sealing tests on the plurality of battery cells using a non-destructive inspection method.

Additionally, according to the embodiment of the present disclosure, it is possible to obtain the advantageous effect of simplifying the process of inspecting the sealing performance of a battery cell and improving the accuracy and reliability of the inspection.

Additionally, according to the embodiment of the present disclosure, it is possible to obtain the advantageous effect of enabling an operator to quickly and accurately inspect whether a battery cell leaks on the site without using expensive equipment or relying heavily on the experience and capability of the operator.

Additionally, according to the embodiment of the present disclosure, it is possible to obtain the advantageous effect of shortening the time required to test the sealing performance of a battery cell and reducing the cost.

### BRIEF DESCRIPTION OF THE DRAWINGS

FIG. 1 is a perspective view for describing an apparatus for inspecting a battery cell according to an embodiment of the present disclosure.
FIG. 2 is an exploded perspective view for describing an apparatus for inspecting a battery cell according to an embodiment of the present disclosure.
FIG. 3 is an exploded cross-sectional view for describing an apparatus for inspecting a battery cell according to an embodiment of the present disclosure.
FIG. 4 is an enlarged view of the "A" part of FIG. 3.
FIG. 5 is a view for describing a sealing member as an apparatus for inspecting a battery cell according to an embodiment of the present disclosure.
FIG. 6 is an enlarged view of the "B" part of FIG. 5.
FIG. 7 is a view for describing a battery cell tray as an apparatus for inspecting a battery cell according to an embodiment of the present disclosure.

### DETAILED DESCRIPTION

Hereinafter, preferred embodiments of the present disclosure will be described in detail with reference to the accompanying drawings.

However, the technical idea of the present disclosure is not limited to some of the embodiments described, but may be implemented in various different forms, and within the scope of the technical idea of the present disclosure, one or more of the components may be selectively combined or substituted between the embodiments.

Additionally, the terms (including technical and scientific terms) used in the embodiments of the present disclosure may be construed as meanings generally understood by those having ordinary skill in the art to which the present disclosure pertains, unless explicitly specifically defined and described, and terms that are commonly used, such as terms defined in dictionaries, may be construed in consideration of the contextual meaning of the related technology.

Additionally, the terms used in the embodiments of the present disclosure are for the purpose of describing the embodiments and are not intended to limit the present disclosure.

In this specification, the singular form may include the plural form unless specifically stated in the phrase, and when described as "at least one (or more than one) of A, B, and C", it may include one or more of all combinations that can be combined with A, B, and C.

Additionally, in describing the components of the embodiments of the present disclosure, terms such as first, second, A, B, (a), (b), and the like may be used.

These terms are only intended to distinguish the components from other components, and do not limit the nature, order, or sequence of the corresponding components.

And, when a component is described as being 'connected', 'coupled', or 'contacted' to another component, this may include not only cases where the component is directly connected, coupled, or contacted to another component, but also cases where the component is 'connected', 'coupled', or 'contacted' to another component through a third component.

Additionally, when it is described as being formed or disposed "on top of (above) or on bottom of (below)" each component, the top (above) or bottom (below) may include not only cases where two components are in direct contact with each other, but also cases where one or more other components are formed or disposed between the two components. Additionally, when expressed as "on top of (above) or on bottom of (below)", this may include the meaning of not only the upward direction but also the downward direction based on one component.

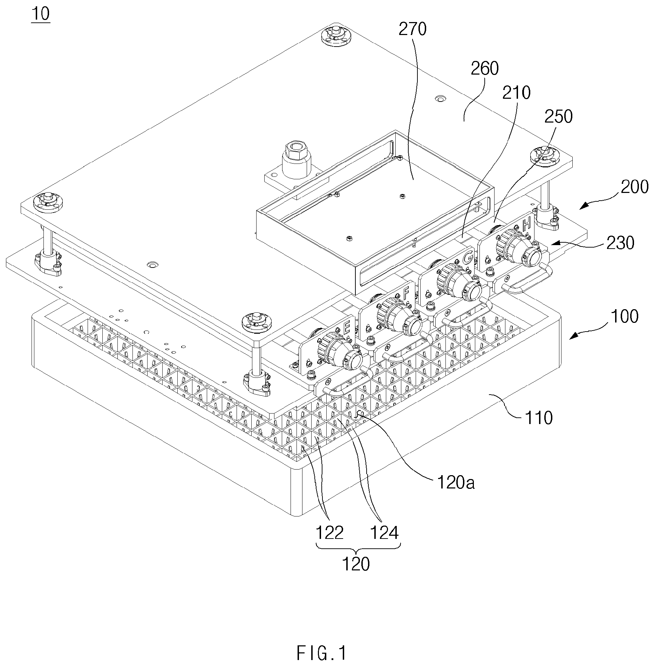

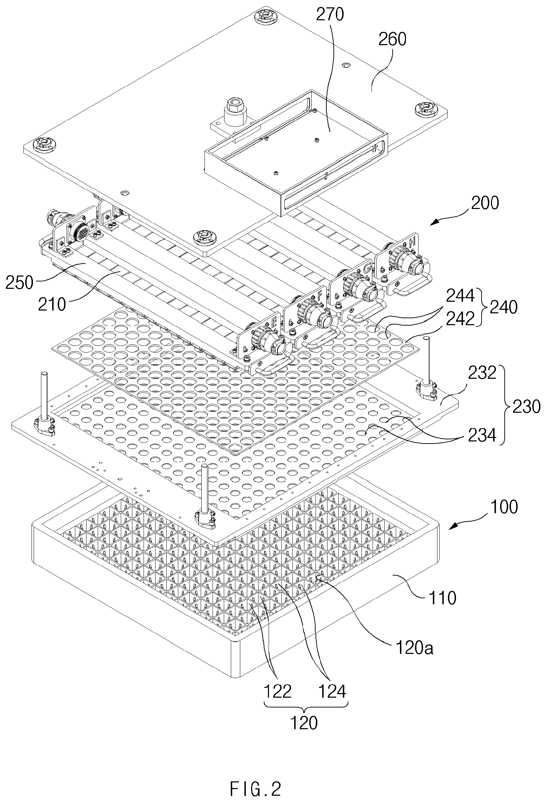

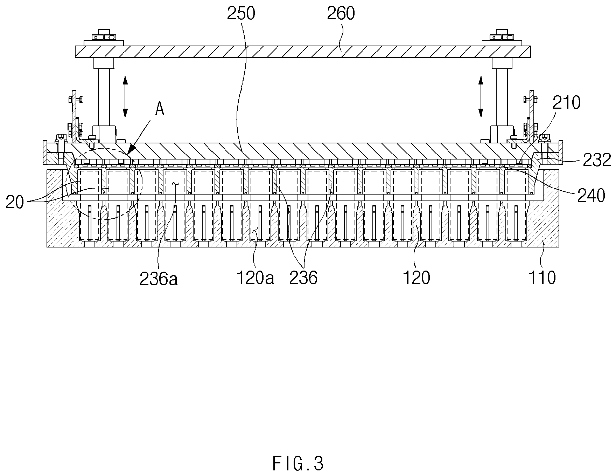

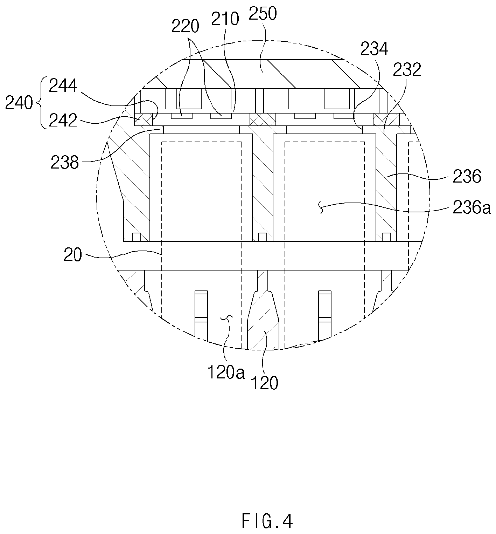

Referring to FIGS. 1 to 7, an apparatus for inspecting a battery cell 10 according to an embodiment of the present disclosure includes a battery cell tray 100 in which a plurality of battery cells 20 are seated, and a sensing unit 200 that simultaneously senses leak gas leaking from the plurality of battery cells 20 individually for each battery cell 20.

For reference, the apparatus for inspecting a battery cell 10 according to an embodiment of the present disclosure may be used to inspect the sealing performance (leakage) of various battery cells 20 according to required conditions and design specifications, and the present disclosure is not limited or restricted by the type and structure of the battery cell 20 used in the apparatus for inspecting a battery cell 10.

According to a preferred embodiment of the present disclosure, the battery cell 20 may include an electrode assembly (not shown) and a case (exterior material) (not shown) that accommodates the electrode assembly therein.

According to a preferred embodiment of the present disclosure, the electrode assembly may include an electrode and a separator stacked on the electrode.

Hereinafter, an example in which the electrode assembly is wound around one end in the form of a jelly roll will be described. According to another embodiment of the present disclosure, it is also possible to form an electrode assembly by stacking a plurality of unit cells having a roughly flat plate shape.

For example, the electrode may include a first electrode stacked on one surface (e.g., top surface) of the separator, and a second electrode stacked on the other surface (e.g., bottom surface) of the separator.

Hereinafter, an example in which the first electrode is configured as a positive electrode and the second electrode is configured as a negative electrode will be described. According to another embodiment of the present disclosure, it is also possible to configure the first electrode as a negative electrode and the second electrode as a positive electrode.

The first electrode (positive electrode) and the second electrode (negative electrode) may be formed by applying a slurry obtained by mixing an electrode active material, a binder, and a plasticizer to a positive electrode current collector and a negative electrode current collector.

The positive electrode current collector is not particularly limited as long as it has conductivity without causing a chemical change in the battery. For example, stainless steel, aluminum, nickel, titanium, calcined carbon, or aluminum or stainless steel surface-treated with carbon, nickel, titanium, silver, or the like may be used as the positive electrode current collector.

The negative electrode current collector is not particularly limited as long as it has conductivity without causing a chemical change in the battery. For example, copper, stainless steel, aluminum, nickel, titanium, calcined carbon, or aluminum or stainless steel surface-treated with carbon, nickel, titanium, silver, or the like may be used as the negative electrode current collector. For example, a thin copper film (e.g., electrolytic copper foil or pressure copper foil) may be used as the negative electrode current collector.

The separator is designed to separate the negative electrode and the positive electrode and provide a passage for lithium ions to move, and may be used without any particular limitation as long as it is generally used as a separator in a lithium secondary battery.

For example, as a separator, a porous polymer film, for example, a porous polymer film made of a polyolefin-based polymer such as an ethylene homopolymer, a propylene homopolymer, an ethylene/butene copolymer, an ethylene/hexene copolymer, and an ethylene/methacrylate copolymer, or a stacked structure of two or more layers thereof may be used.

Alternatively, a conventional porous nonwoven fabric, for example, a nonwoven fabric made of high-melting-point glass fibers, polyethylene terephthalate fibers, or the like may be used as a separator, or a coated separator including a ceramic component or a polymer material may be used to ensure heat resistance or mechanical strength.

The case accommodates the electrode assembly therein and is provided to completely surround the circumference of the electrode assembly.

The case may be provided in various structures capable of accommodating the electrode assembly therein, and the present disclosure is not limited or restricted by the structure and shape of the case.

For example, the case may include a case body (not shown) having a roughly hollow cylindrical shape, and a cap (not shown) sealing an opening of the case body.

According to another embodiment of the present disclosure, the case may be configured to have a rectangular block shape (e.g., a rectangular can) or other shapes.

The case may be formed of various materials according to required conditions and design specifications, and the present disclosure is not limited or restricted by the type and characteristics of the material of the case.

For example, the case may be configured to have a structure including a metal layer, such as an aluminum thin film or the like, in order to protect components such as an electrode assembly and an electrolyte injected into the case and to enhance supplementation and heat dissipation with respect to electrochemical properties by the electrode assembly and the electrolyte.

For reference, the electrolyte solution (electrolyte) used in the present disclosure may include various electrolytes that may define a passage for lithium ions to move in a lithium secondary battery, for example, an organic liquid electrolyte, an inorganic liquid electrolyte, a solid polymer electrolyte, a gel-type polymer electrolyte, a solid inorganic electrolyte, a molten inorganic electrolyte, and the like, and the present disclosure is not limited or restricted by the type and characteristics thereof. For example, the electrolyte may include an organic solvent and a lithium salt. Any organic solvent may be used without particular limitation as long as it may serve as a medium through which ions involved in the electrochemical reaction of the battery may move.

Referring to FIGS. 1 to 3 and FIG. 7, the battery cell tray 100 is provided to transfer a plurality of battery cells 20 in an inline manner.

For example, the battery cell tray 100 in which a plurality of battery cells 20 are seated may be transferred along a preset movement path (e.g., a linear movement path) via a conventional conveyor.

The battery cell tray 100 may be provided in various structures in which a plurality of battery cells 20 may be seated, and the present disclosure is not limited or restricted by the structure of the battery cell tray 100.

According to a preferred embodiment of the present disclosure, the battery cell tray 100 may include a tray body 110, and a partition portion 120 provided in the tray body 110 and dividing the internal space of the tray body 110 into a plurality of seating spaces 120a where a plurality of battery cells 20 are individually seated.

The tray body 110 may be provided in various structures having an internal space, and the present disclosure is not limited or restricted by the structure and shape of the tray body 110.

For example, the tray body 110 may be formed in a roughly rectangular box shape with an open top. According to another embodiment of the present disclosure, the tray body may be formed in a cylindrical shape or other shapes.

The partition portion 120 is provided to divide the internal space of the tray body 110 into a plurality of independently partitioned seating spaces 120a.

The partition portion 120 may be provided in various structures capable of dividing the internal space of the tray body 110 into a plurality of seating spaces 120a, and the present disclosure is not limited or restricted by the structure of the partition portion 120 and the number and shape of the seating holes.

According to a preferred embodiment of the present disclosure, the partition portion 120 may include a first partition member 122 dividing the internal space along a first direction of the tray body 110, and a second partition member 124 dividing the internal space along a second direction intersecting the first direction and defining the seating space 120a in cooperation with the first partition member 122.

For example, the first partition member 122 and the second partition member 124 may be formed to have a roughly rectangular plate shape, and the first partition member 122 and the second partition member 124 may be disposed to be orthogonal to each other and cooperatively form a roughly lattice shape.

Hereinafter, an example in which a plurality of seating spaces 120a having a roughly rectangular cross-sectional shape are disposed in an n×n matrix via the first partition member 122 and the second partition member 124 inside the tray body 110 will be described. For example, the internal space of the tray body 110 may be divided into a total of 256 seating spaces 120a to form a 16×16 matrix by the partition portion 120. According to another embodiment of the present disclosure, the partition portion may divide the internal space of the tray body into fewer than or more than 256 seating spaces.

The lower end of the battery cell 20 may be individually accommodated in each seating space 120a, and the upper end of each battery cell 20 may be exposed to the upper part of the tray body 110 in a state where each battery cell 20 is accommodated in the seating space 120a.

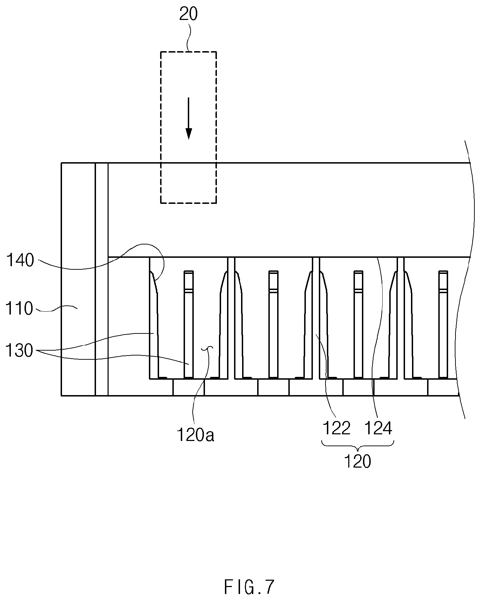

Referring to FIG. 7, according to a preferred embodiment of the present disclosure, the apparatus for inspecting a battery cell 10 may include a support rib 130 provided on an inner wall surface of the seating space 120a and supporting the battery cell 20 with respect to the tray body 110.

The support rib 130 is provided to suppress the movement and shaking of the battery cell 20 accommodated in the seating space 120a and to stably maintain the arrangement state of the battery cell 20.

The support rib 130 may be provided in various structures capable of supporting the battery cell 20 with respect to the tray body 110, and the present disclosure is not limited or restricted by the structure and shape of the support rib 130.

For example, the support rib 130 may be formed on the inner wall surface (surfaces of the first partition member and the second partition member) of the seating space 120a to have a roughly linear shape along the upper-lower direction. According to another embodiment of the present disclosure, the support rib may be formed in a curved shape or other protruding shape.

According to a preferred embodiment of the present disclosure, the apparatus for inspecting a battery cell 10 may include a slope guide portion 140 provided at an upper end of the support rib 130 and guiding a lower end of the battery cell 20 to the seating space 120a.

For example, the slope guide portion 140 is provided to form an entry part (an entry part where the battery cell enters the interior of the buffer space) having a cross-sectional area that gradually expands along a direction from the bottom to the top of the seating space 120a.

The slope guide portion 140 may be formed in a flat or curved shape, and the present disclosure is not limited or restricted by the structure and shape of the slope guide portion 140.

In this way, according to the embodiment of the present disclosure, the slope guide portion 140 may be provided at the upper end of the support rib 130, thereby allowing the battery cell 20 to enter the interior of the seating space 120a more smoothly.

Additionally, according to the embodiment of the present disclosure, the lower end of the battery cell 20 is guided into the guide space along the slope guide portion 140, so that even if the center of the battery cell 20 and the center of the buffer space are misaligned, the center of the battery cell 20 may be coaxially guided (aligned) to the center of the buffer space, thereby minimizing misinsertion and misalignment of the battery cell 20 and obtaining the advantageous effect of improving inspection efficiency.

Referring to FIGS. 1 to 4, the sensing unit 200 is provided to simultaneously sense leak gas (e.g., dimethyl carbonate, DMC) leaking from a plurality of battery cells 20 individually for each battery cell 20.

According to a preferred embodiment of the present disclosure, the sensing unit 200 is configured to sense the leak gas in an atmospheric pressure environment.

In this way, according to the embodiment of the present disclosure, the sensing unit 200 is configured to sense the leak gas in an atmospheric pressure environment, so that it is not necessary to additionally provide a separate vacuum chamber and equipment for forming a vacuum environment, thereby obtaining the advantageous effect of simplifying the structure and inspection process and reducing the cost.

The sensing unit 200 may be provided in various structures capable of individually and simultaneously sensing the leak gas leaking from each battery cell 20, and the present disclosure is not limited or restricted by the structure of the sensing unit 200.

According to a preferred embodiment of the present disclosure, the sensing unit 200 may include a sensor board 210, a sensing element 220 provided on the sensor board 210 and sensing the leak gas, and a leak gas guide 230 provided between the sensor board 210 and the battery cell tray 100 and individually guiding the leak gas leaking from the battery cell 20 to the sensing element 220.

The sensor board 210 is a base substrate for mounting the sensing element 220, and a conventional printed circuit board (PCB) may be used.

The sensor board 210 may be provided to have various structures and sizes according to required conditions and design specifications, and the present disclosure is not limited or restricted by the structure and size of the sensor board 210.

For example, the sensor board 210 may be provided by combining (arranging) a plurality of PCBs. According to another embodiment of the present disclosure, it is also possible to configure the sensor board using only one PCB.

The sensing element 220 is provided to individually sense the leak gas leaking from each battery cell 20.

As the sensing element 220, various sensors capable of sensing the leak gas leaking from the battery cell 20 may be used, and the present disclosure is not limited or restricted by the type and characteristics of the sensing element 220.

For example, a sensor (e.g., a semiconductor gas sensor) whose resistance varies due to an oxidation-reduction reaction by a leak gas (e.g., DMC) may be used as the sensing element 220.

Hereinafter, an example in which three sensing elements 220 form a sensing element group and a plurality of sensing element groups are configured to individually sense leak gas leaking from each battery cell 20 will be described. According to another embodiment of the present disclosure, the sensing element group may be configured to include two or fewer sensing elements or four or more sensing elements. Alternatively, it is also possible to configure a single sensing element to sense leak gas leaking from each corresponding battery cell.

The leak gas guide 230 may be provided in various structures capable of individually guiding leak gas leaking from each battery cell 20 to the sensing element 220 (sensing element group), and the present disclosure is not limited or restricted by the structure of the leak gas guide 230.

According to a preferred embodiment of the present disclosure, the leak gas guide 230 may include a guide plate 232 provided between the sensing element 220 and the battery cell tray 100, and a guide hole 234 formed through the guide plate 232 and individually guiding the leak gas to the sensing element 220 for each battery cell 20.

For example, the guide plate 232 may be provided to have a roughly rectangular plate shape corresponding to the battery cell tray 100.

The guide hole 234 is formed through the guide plate 232 to correspond one-to-one to each battery cell 20.

The guide hole 234 may be provided in various structures according to required conditions and design specifications, and the present disclosure is not limited or restricted by the structure and shape of the guide hole 234.

For example, the guide hole 234 may be formed in a roughly circular hole shape. According to another embodiment of the present disclosure, the guide hole may be configured to have a rectangular or other shape.

According to a preferred embodiment of the present disclosure, the apparatus for inspecting a battery cell 10 may include a guide rib 236 provided on a bottom surface of the guide plate 232 facing the battery cell tray 100 and defining a receiving space 236a in which an upper end of the battery cell 20 is individually accommodated.

The guide rib 236 may be provided in various structures capable of defining a receiving space 236a communicating with the guide hole 234, and the present disclosure is not limited or restricted by the structure and shape of the guide rib 236.

For example, the guide rib 236 may be formed to have a roughly hollow cylindrical shape. According to another embodiment of the present disclosure, the guide rib may be configured to have a rectangular cross-sectional or other cross-sectional shape.

In this way, according to the embodiment of the present disclosure, a guide rib 236 is provided on the bottom surface of the guide plate 232, and the upper end of the battery cell 20 is accommodated in the receiving space 236a of the guide rib 236, so that the leak gas leaking from the battery cell 20 may be concentrated into the guide hole 234 without being scattered to the surroundings, thereby obtaining the advantageous effect of further improving the sensing performance and accuracy of the leak gas.

According to a preferred embodiment of the present disclosure, the apparatus for inspecting a battery cell 10 may include a stopper portion 238 provided on the guide plate 232 and supporting the battery cell 20 to be spaced apart from the sensing element 220.

This is because, when the electrolyte leaking from the battery cell 20 is in direct contact with the sensing element 220, the sensing element 220 becomes contaminated by the electrolyte, making it difficult for the sensing element 220 to perform the inspection process normally.

However, according to the embodiment of the present disclosure, a stopper portion 238 is provided on the guide plate 232, and the battery cell 20 is supported to be spaced apart from the sensing element 220 via the stopper portion 238, so that even if the electrolyte leaks from the battery cell 20, the electrolyte may be prevented from directly contacting the sensing element 220, thereby obtaining the advantageous effect of minimizing the deterioration of inspection accuracy and reliability due to contamination of the sensing element 220.

The stopper portion 238 may be provided in various structures capable of supporting the battery cell 20 to be spaced apart from the sensing element 220, and the present disclosure is not limited or restricted by the structure of the stopper portion 238.

According to a preferred embodiment of the present disclosure, the stopper portion 238 may be configured to support the edge end of the battery cell 20 along the circumferential direction of the battery cell 20.

For example, the stopper portion 238 may be formed in a protruding shape on the inner wall surface of the upper end of the receiving space 236a, and the guide hole 234 may be formed inside the stopper portion 238.

In this way, when the upper end of the battery cell 20 is supported by the stopper portion 238, a predetermined gap may be secured between the battery cell 20 and the sensing element 220, so that even if the electrolyte leaks from the battery cell 20, the electrolyte may be prevented from directly contacting the sensing element 220.

According to a preferred embodiment of the present disclosure, the apparatus for inspecting a battery cell 10 may include a gasket member 240 provided between the sensor board 210 and the leak gas guide 230 and independently sealing the sensing element 220 corresponding to the battery cell 20.

The gasket member 240 is provided to independently seal the sensing element 220 (or sensing element group) between the sensor board 210 and the leak gas guide 230 so that the leak gas passing through the guide hole 234 moves only to the corresponding sensing element 220 (or sensing element group) and does not move to other sensing elements 220 in the vicinity.

The gasket member 240 may be provided in various structures capable of independently sealing the sensing element 220 (or sensing element group) between the sensor board 210 and the leak gas guide 230, and the present disclosure is not limited or restricted by the structure of the gasket member 240.

According to a preferred embodiment of the present disclosure, the gasket member 240 may include a gasket sheet 242 interposed between the sensor board 210 and the guide plate 232, and a through hole 244 formed through the gasket sheet 242 to communicate with the guide hole.

For example, the gasket sheet 242 may be formed in a roughly rectangular sheet shape, and the through hole 244 may be formed to have a roughly circular cross-sectional shape.

The gasket member 240 may be made of a conventional elastic material such as rubber, silicone, Teflon, or the like, and the present disclosure is not limited or restricted by the material and structure of the gasket member 240.

In this way, according to the embodiment of the present disclosure, each sensing element 220 (or sensing element group) may be independently sealed via the gasket member 240, thereby obtaining the advantageous effect of minimizing malfunction (false sensing) of the sensing element 220 due to leak gas introduced from other guide holes 234 in the vicinity and improving stability and reliability.

Meanwhile, in the above-described and illustrated embodiment of the present disclosure, an example in which the gasket member 240 is composed of a single gasket sheet 242 is described, but according to another embodiment of the present disclosure, it is also possible to configure the gasket member by connecting or combining a plurality of gasket sheets.

Additionally, in the above-described and illustrated embodiments of the present disclosure, an example in which a gasket member 240 is provided between the sensor board 210 and the guide plate 232 to independently seal each sensing element 220 (or sensing element group) is described, but according to another embodiment of the present disclosure, it is also possible to form a separate structure (e.g., a cylindrical rib) on the bottom surface of the sensor board (or the top surface of the guide plate) to independently seal each sensing element (or sensing element group), in place of the gasket member.

According to a preferred embodiment of the present disclosure, the apparatus for inspecting a battery cell 10 may include a sensor plate 250 supporting the sensor board 210, wherein the sensor plate 250 may be seated on the guide plate 232.

The sensor plate 250 may be provided in various structures capable of supporting the sensor board 210, and the present disclosure is not limited or restricted by the structure of the sensor plate 250.

For example, the sensor plate 250 may be configured by connecting a plurality of straight plate members. Alternatively, it is also possible to configure the sensor plate 250 with a single plate member.

The sensor plate 250 may be stacked (seated) on top of the guide plate 232, and a predetermined gap may be secured between the sensing element 220 and the guide hole 234 in a state where the sensor plate 250 is seated on the guide plate 232.

In this way, according to the embodiment of the present disclosure, the sensor board 210 may be supported by the sensor plate 250, thereby obtaining the advantageous effect of more stably supporting the arrangement state of the sensor board 210 with respect to the guide plate 232 while ensuring the structural rigidity of the sensor board 210.

According to a preferred embodiment of the present disclosure, the apparatus for inspecting a battery cell 10 may include a support member 260 provided above the sensing unit 200 and selectively supporting the sensing unit 200 to allow it to move up and down.

The support member 260 may be provided in various structures capable of selectively supporting the sensing unit 200 to allow it to move up and down, and the present disclosure is not limited or restricted by the structure and shape of the support member 260.

For example, the support member 260 may be formed to have a roughly rectangular plate shape, and the guide plate 232 of the sensing unit 200 may be selectively suspended from the support member 260 via a guide rod (not shown) to allow it to move up and down.

The vertical movement of the sensing unit 200 with respect to the support member 260 may be implemented in various ways according to required conditions and design specifications.

For example, the vertical movement of the sensing unit 200 with respect to the support member 260 may be implemented by a conventional linear motion system such as a motor, a solenoid, or a linear motor. According to another embodiment of the present disclosure, it is also possible to configure the sensing unit to move up and down with respect to the support member in response to the rotation of the lead screw.

For reference, when the battery cell tray 100 in which a plurality of battery cells 20 are seated is transferred to the lower part of the sensing unit 200, the sensing unit 200 may descend in a downward direction (in a direction approaching the battery cell tray) to perform a leak test for each battery cell 20 for a preset time (e.g., within 40 seconds).

When the inspection for each battery cell 20 is completed, the sensing unit 200 may move upward again, and the battery cell tray 100 may be transferred along a preset movement path.

According to a preferred embodiment of the present disclosure, the apparatus for inspecting a battery cell 10 may include a control board 270 provided on the support member 260 and outputting sensing values sensed by the sensing unit 200 for each battery cell 20 to the outside.

The control board 270 may collect and calculate the sensing values sensed by each sensing element 220 and transmit them to an external controller or storage.

The control board 270 may include a central processing unit (CPU) or a semiconductor device that executes processing for instructions stored in a memory and/or storage. The memory and storage may include various types of volatile or nonvolatile storage media. For example, the memory may include a read only memory (ROM) and/or a random access memory (RAM).

The control board 270 may be mounted at various points of the support member 260 according to required conditions and design specifications, and the present disclosure is not limited or restricted by the mounting point of the control board 270.

For example, a receiving box (not shown) may be provided on the upper surface of the support member 260, and the control board 270 may be accommodated inside the receiving box.

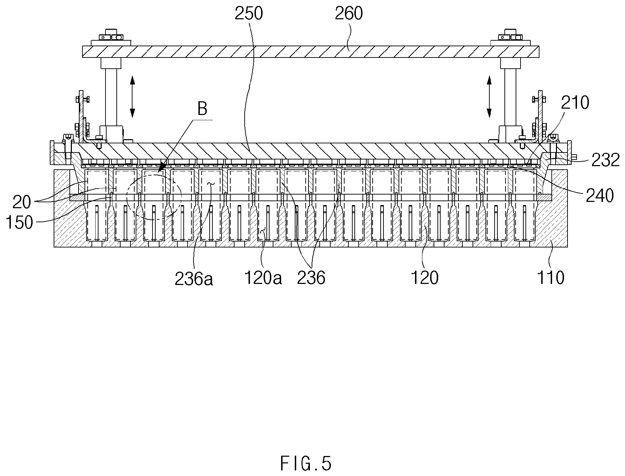

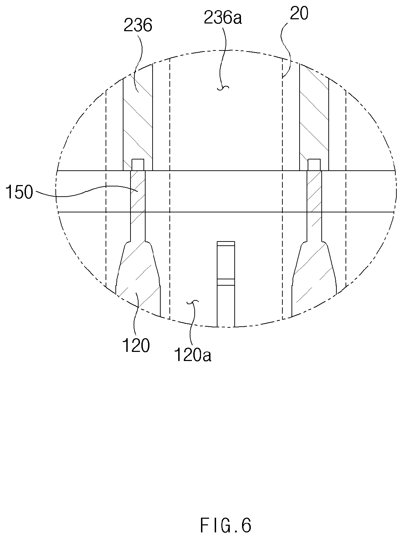

Referring to FIGS. 5 and 6, according to a preferred embodiment of the present disclosure, the apparatus for inspecting a battery cell 10 may include a sealing member 150 provided between the guide rib 236 and the partition portion 120 and sealing the receiving space 236a and the seating space 120a for each battery cell 20.

The sealing member 150 is provided to suppress the leakage of leak gas leaking from the battery cell 20 into the gap between the receiving space 236a and the seating space 120a (the gap between the guide rib and the partition portion), while suppressing the flow of contaminated air or gas from the outside into the gap between the receiving space 236a and the seating space 120a.

The sealing member 150 may be provided in various structures capable of sealing the gap between the guide rib 236 and the partition portion 120, and the present disclosure is not limited or restricted by the structure of the sealing member 150.

For example, the sealing member 150 is formed in a roughly rectangular sheet shape, and may include a sealing sheet (not shown) interposed between the guide rib 236 and the partition portion 120, and a connection hole (not shown) formed in the sealing sheet to communicate with the receiving space 236a and the seating space 120a.

The sealing member 150 may be made of a conventional elastic material such as rubber, silicone, Teflon, or the like, and the present disclosure is not limited or restricted by the material and structure of the sealing member 150.

In this way, according to the embodiment of the present disclosure, the gap between the guide rib 236 and the partition portion 120 may be sealed via the sealing member 150, so that it is possible to block the leakage of leak gas leaking from the battery cell 20 into the gap between the receiving space 236a and the seating space 120a (the gap between the guide rib and the partition portion) or the flow of contaminated air or gas from the outside into the gap between the receiving space 236a and the seating space 120a, thereby obtaining the advantageous effect of minimizing the deterioration of the inspection accuracy and reliability of the sensing element 220 due to the detection amount of leak gas falling below a reference value or the inflow of contaminated air.

Although the present disclosure has been described centered on the embodiment hereinabove, this is merely an example and does not limit the present disclosure, and those having ordinary skill in the art will understand that various modifications and applications not exemplified above are possible without departing from the essential characteristics of the present embodiment. For example, each component specifically shown in the embodiment may be modified and implemented. And, the differences related to such modifications and applications should be construed as being included in the scope of the present disclosure defined in the appended claims.

### [List of Reference Numerals]

10: Apparatus for inspecting battery cell
20: Battery cell
100: Battery cell tray
110: Tray body
120: Partition portion
120a: Seating space
122: First partition member
124: Second partition member
130: Support rib
140: Slope guide portion
150: Sealing member
200: Sensing unit
210: Sensor board
220: Sensing element
230: Leak gas guide
232: Guide plate
234: Guide hole
236: Guide rib
236a: Receiving space
238: Stopper portion
240: Gasket member
242: Gasket sheet
244: Through hole
250: Sensor plate
260: Support member
270: Control board

## Claims

1. An apparatus for inspecting a battery cell comprising:
a battery cell tray in which a plurality of battery cells are seated; and
a sensing unit that simultaneously senses leak gas leaking from the plurality of battery cells individually for each battery cell.

2. The apparatus for inspecting a battery cell according to claim 1,
wherein the sensing unit comprises:
a sensor board;
a sensing element provided on the sensor board and sensing the leak gas; and
a leak gas guide provided between the sensor board and the battery cell tray and individually guiding the leak gas leaking from the battery cell to the sensing element.

3. The apparatus for inspecting a battery cell according to claim 2,
wherein the leak gas guide comprises:
a guide plate provided between the sensing element and the battery cell tray; and
a guide hole formed through the guide plate and individually guiding the leak gas to the sensing element for each battery cell.

4. The apparatus for inspecting a battery cell according to claim 3, comprising:
a guide rib provided on a bottom surface of the guide plate facing the battery cell tray and defining a receiving space in which an upper end of the battery cell is individually accommodated.

5. The apparatus for inspecting a battery cell according to claim 3, comprising:
a stopper portion provided on the guide plate and supporting the battery cell to be spaced apart from the sensing element.

6. The apparatus for inspecting a battery cell according to claim 5,
wherein the stopper portion supports the edge end of the battery cell along the circumferential direction of the battery cell.

7. The apparatus for inspecting a battery cell according to claim 3, comprising:
a gasket member provided between the sensor board and the leak gas guide and independently sealing the sensing element corresponding to the battery cell.

8. The apparatus for inspecting a battery cell according to claim 7,
wherein the gasket member comprises:
a gasket sheet interposed between the sensor board and the guide plate; and
a through hole formed through the gasket sheet to communicate with the guide hole.

9. The apparatus for inspecting a battery cell according to claim 3, comprising:
a sensor plate supporting the sensor board,
wherein the sensor plate is seated on the guide plate.

10. The apparatus for inspecting a battery cell according to claim 1, comprising:
a support member provided above the sensing unit and selectively supporting the sensing unit to allow it to move up and down.

11. The apparatus for inspecting a battery cell according to claim 10, comprising:
a control board provided on the support member and outputting sensing values sensed by the sensing unit for each battery cell to the outside.

12. The apparatus for inspecting a battery cell according to claim 1,
wherein the sensing unit senses the leak gas in an atmospheric pressure environment.

13. The apparatus for inspecting a battery cell according to claim 4,
wherein the battery cell tray comprises:
a tray body; and
a partition portion provided in the tray body and dividing an internal space of the tray body into a plurality of seating spaces where the plurality of battery cells are individually seated.

14. The apparatus for inspecting a battery cell according to claim 13,
wherein the partition portion comprises:
a first partition member dividing the internal space along a first direction of the tray body; and
a second partition member dividing the internal space along a second direction intersecting the first direction and defining the seating space in cooperation with the first partition member.

15. The apparatus for inspecting a battery cell according to claim 13, comprising:
a support rib provided on an inner wall surface of the seating space and supporting the battery cell with respect to the tray body.

16. The apparatus for inspecting a battery cell according to claim 15, comprising:
a slope guide portion provided at an upper end of the support rib and guiding a lower end of the battery cell to the seating space.

17. The apparatus for inspecting a battery cell according to claim 13, comprising:
a sealing member provided between the guide rib and the partition portion and sealing the receiving space and the seating space for each battery cell.
